# EUROPÄISCHE PATENTSCHRIFT

(11) **EP 0 696 916 B1**
(45) Veröffentlichungstag und Bekanntmachung des Hinweises auf die Patenterteilung: **22.10.1997**
(21) Anmeldenummer: 94914405.9
(22) Anmeldetag: 19.04.1994
(51) Int. Cl.: A61K 7/06

(54) **HAARFESTIGUNGSMITTEL**
HAIR SETTING AGENT
AGENT DE FIXATION CAPILLAIRE

(30) Priorität: 30.04.1993 DE 4314305
(43) Veröffentlichungstag der Anmeldung: 21.02.1996
(73) Patentinhaber: BASF AKTIENGESELLSCHAFT, 67056 Ludwigshafen (DE)
(72) Erfinder: SCHEHLMANN, Volker, D-67254 Römerberg (DE); SPERLING-VIETMEIER, Karin, D-67433 Neustadt (DE); SANNER, Axel, D-67227 Frankenthal (DE); BLANKENBURG, Rainer, D-67067 Ludwigshafen (DE)
(86) Internationale Anmeldenummer: EP9401205
(87) Internationale Veröffentlichungsnummer: WO9424986

(56) Entgegenhaltungen:
- EP-A- 0 373 442
- EP-A- 0 379 082
- EP-A- 0 480 280
- FR-A- 2 424 738
- US-A- 4 842 852
- US-A- 4 874 604

## Beschreibung

Die vorliegende Erfindung betrifft neue Haarfestigungsmittel, welche als Filmbildner Copolymerisate auf der Basis von tert.-Butylacrylat oder tert.-Butylmethacrylat mit einem K-Wert von 10 bis 50 enthalten, die durch radikalische Polymerisation von
A) 30 bis 72 Gew.-% tert.-Butylacrylat oder tert.-Butylmethacrylat oder einer Mischung hieraus als Monomerem A,
B) 10 bis 28 Gew.-% Acrylsäure oder Methacrylsäure oder einer Mischung hieraus als Monomerem B und
C) 0 bis 60 Gew.-% eines radikalisch copolymerisierbaren Monomeren oder einer radikalisch copolymerisierbaren Monomerenmischung als Monomeren C, wobei mindestens eines der Monomeren C ein Homopolymerisat mit einer Glastemperatur kleiner als 30°C liefert,
erhältlich sind, wobei die Carboxylgruppen der Copolymerisate teilweise oder vollständig neutralisiert sind.

Aus der EP-A 379 082 sind Haarfestigungsmittel bekannt, welche als Filmbildner Copolymerisate auf der Basis von tert.-Butylacrylat und/oder tert.-Butylmethacrylat mit einem K-Wert von 10 bis 50 enthalten, die durch radikalische Polymerisation von
A) 75 bis 99 Gew.-% tert.-Butylacrylat und/oder tert.-Butylmethacrylat,
B) 1 bis 25 Gew.-% Acrylsäure und/oder Methacrylsäure und
C) 0 bis 10 Gew.-% eines weiteren radikalisch copolymerisierbaren Monomeren
erhältlich sind, wobei die Carboxylgruppen der Copolymerisate wie üblich teilweise oder vollständig durch Amine neutralisiert sind.

Noch verbesserungsbedürftig bei diesen aus dem Stand der Technik bekannten Mitteln sind jedoch die Auswaschbarkeit des Polymeren aus dem Haar, die Löslichkeit des Filmbildners in wasserhaltigen Zubereitungen, die heute immer mehr die auf reinen organischen Lösungsmitteln basierenden und deshalb ökologisch bedenklichen Zubereitungen verdrängen, sowie Griff und Festigung des behandelten Haars, da sich mit den Haarfestigungsmitteln des Standes der Technik behandeltes Haar meist zu spröde oder zu rauh anfühlt.

Aufgabe der vorliegenden Erfindungen war es demnach, ein Haarfestigungsmittel bereitzustellen, welches die oben geschilderten Nachteile nicht mehr aufweist.

Demgemäß wurde das eingangs definierte Haarfestigungsmittel gefunden.

Das bzw. die Monomere C dienen zur Modifikation der Eigenschaften des Copolymerisates. In einer bevorzugten Ausführungsform werden hierzu C₁- bis C₁₈-Alkylacrylate oder -methacrylate, insbesondere C₁- bis C₄-Alkylacrylate oder -methacrylate, oder eine Mischung aus diesen (Meth)acrylaten mit N-C₁- bis -C₁₈-Alkylacrylamiden oder -methacrylamiden, insbesondere N-C₁- bis -C₄-Alkylacrylamiden oder -methacrylamiden, eingesetzt. Als C₁- bis C₄-Alkylreste in den genannten (Meth)Acrylaten und (Meth)Acrylamiden kommen Methyl, Ethyl, n-Propyl, iso-Propyl, n-Butyl, iso-Butyl, sec.-Butyl und tert.-Butyl in Betracht.

Man erhält besonders gute Ergebnisse, wenn als Monomere C unverzweigte C₂- bis C₄-Alklacrylate allein oder in Mischung mit verzweigten N-C₃- bis -C₄-Alkylacrylamiden eingesetzt werden. Besonders bevorzugt werden hierfür Ethylacrylat oder eine Mischung aus Ethylacrylat und N-tert.-Butylacrylamid.

In einer bevorzugten Ausführungsform beträgt die Zusammensetzung des als Filmbildners verwendeten Copolymerisates
A) 50 bis 72 Gew.-%, insbesondere 60 bis 70 Gew.-% des Monomeren A,
B) 12 bis 25 Gew.-%, insbesondere 15 bis 23 Gew.-% des Monomeren B und
C) 3 bis 38 Gew.-%, insbesondere 7 bis 25 Gew.-% des Monomeren C.

Die Obergrenze des Monomeren A von 72 Gew.-% ist besonders kritisch, da darüber hinaus die vorteilhaften Eigenschaften des erfindungsgemäßen Haarfestigungsmittels nicht mehr feststellbar sind. Der deutliche Wechsel im Eigenschaftsspektrum des Haarfestigungsmittels zwischen einem Gehalt von 72 und 75 Gew.-% Monomer A war insbesondere im Hinblick auf die aus der EP-A 379 082 bekannten Mittel überraschend.

In einer weiteren bevorzugten Ausführungsform ist das als Filmbildner verwendete Copolymerisat aus
B) tert.-Butylacrylat als Monomerem A,
B) Methacrylsäure als Monomerem B und
C) Ethylacrylat oder einer Mischung aus Ethylacrylat und N-tert.-Butylacrylamid als Monomerem C
aufgebaut.

Die beschriebenen Copolymerisate werden in bekannter Weise durch radikalische Copolymerisation der Monomeren A, B und gegebenenfalls C hergestellt. Hierbei arbeitet man nach den üblichen Polymerisationstechniken, zum Beispiel nach den Methoden der Suspensions-, Emulsions- oder Lösungspolymerisation.

Als Initiatoren für die radikalisch ablaufende Polymerisationsreaktion werden die üblichen Peroxo- oder Azoverbindungen, beispielsweise Dibenzoylperoxid, tert.-Butylperpivalat, tert.-Butylper-2-ethylhexanoat, Di-tert.-butylperoxid, tert.-Butylhydroperoxid, 2,5-Dimethyl-2,5-di(tert.-butylperoxy)hexan, Alkalimetall- oder Anmoniumpersulfate, Azo-bis-isobutyronitril, Wasserstoffperoxid oder Redoxinitiatoren zweckmäßigerweise in Mengen von 0,1 bis 2 Gew.-%, bezogen auf das Gewicht der Monomeren, eingesetzt. Man wählt die Mengen an Monomeren und Lösungs- bzw. Dispergiermittel zweckmäßigerweise so, daß man 30 bis 80 gew.-%ige Lösungen bzw. Dispersionen oder Suspensionen der Copolymerisate enthält. Zur Senkung des Restmonomerengehaltes kann eine Nachpolymerisation nach allgemein bekannten Verfahren erfolgen.

Die Copolymerisate sollen K-Werte von 10 bis 60, vorzugsweise 15 bis 40, aufweisen. Der jeweils gewünschte K-Wert läßt sich in an sich bekannter Weise durch Wahl der Polymerisationsbedingungen, beispielsweise der Polymerisationstemperatur und der Initiatorkonzentration, einstellen. Gegebenenfalls, insbesondere bei Anwendung der Emulsions- und Suspenisonspolymerisation, kann der Einsatz von Reglern, insbesondere von Schwefelverbindungen wie Mercaptoethanol, 2-Ethylhexylthioglykolat, Thioglykolsäure oder Dodecylmercaptan zur Reduzierung des K-Wertes angebracht sein. Die K-Werte werden nach Fikentscher, Cellulosechemie, Bd. 13, S. 58 bis 64 (1932) bei 25°C in 1 gew.-%iger ethanolischer Lösung gemessen und stellen ein Maß für das Molgewicht dar.

Derartige Copolymerisate haben üblicherweise Glastemperaturen zwischen 50 und 130°C, insbesondere zwischen 60 und 100°C.

Wird das Haarfestigungsmittel durch Emulsionspolymerisation hergestellt, kann die erhaltene Dispersion entweder direkt in eine wäßrige Haarfestigungszubereitung eingearbeitet werden oder es erfolgt eine Trocknung, z.B. Sprühtrocknung, der Dispersion, so daß das Haarfestigungsmittel als Pulver verwendet und verarbeitet werden kann.

Für die Verwendung in den erfindungsgemäßen Haarfestigungsmitteln werden die Carboxylgruppen der so erhaltenen Copolymerisate üblicherweise mit einem Alkalimetallhydroxid oder vorzugsweise mit einem Amin teilweise oder vollständig, zweckmäßigerweise zu 5 bis 100 %, vorzugsweise zu 30 bis 95 %, neutralisiert. Die Neutralisation erfolgt bevorzugt mit
- einem Mono-, Di- oder Trialkanolamin mit 2 bis 5 C-Atomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 C-Atomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 C-Atomen, beispielsweise N,N-Diethylpropylamin.

Besonders gute Ergebnisse erhielt man mit 2-Amino-2-methylpropanol, Triisopropanolamin und 2-Amino-2-ethylpropan-1,3-diol.

Als Alkalimetallhydroxide eignen sich zur Neutralisation vor allem Natrium- und Kaliumhydroxid.

Die erfindungsgemäßen Haarfestigungsmittel kommen beispielsweise als Haargele, flüssige Haarfestiger, Haarschäume und vor allem als Frisurenfestiger in Form von Sprayzubereitungen zur Anwendung. Besonders bevorzugt werden Haarsprayzubereitungen, die die folgenden Bestandteile enthalten:
- 0,1 bis 20 Gew.-%, vorzugsweise 0,5 bis 10 Gew.-%, insbesondere 2 bis 10 Gew.-% des teilweise oder vollständig neutralisierten Copolymerisates
- 1 bis 99,9 Gew.-%, vorzugsweise 5 bis 50 Gew.-%, insbesondere 10 bis 40 Gew.-% Wasser
- 0 bis 95 Gew.-%, vorzugsweise 20 bis 60 Gew.-%, insbesondere 25 bis 50 Gew.-% eines üblichen organischen Lösungsmittels wie vor allem Ethanol, Isopropanol und Dimethoxymethan und daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische
- 0 bis 90 Gew.-%, vorzugsweise 30 bis 80 Gew.-%, insbesondere 45 bis 70 Gew.-% eines üblichen Treibmittels wie Propan, n-Butan, Isobutan, 2,2-Dimethylbutan, n-Pentan, Isopentan, Dimethylether, Difluorethan, Fluortrichlormethan, Dichlordifluormethan oder Dichlortetrafluorethan oder deren Gemische

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die erfindungsgemäßen Haarfestigungsmittel eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Außerdem können diese Sprayzubereitungen noch geringe Mengen an Parfümölen, beispielsweise 0,1 bis 0,5 Gew.-%, enthalten.

Eine wasserhaltige Standard-Sprayformulierung weist beispielsweise die folgende Zusammensetzung auf:
- 2 bis 10 Gew.-%: des zu 100 % mit 2-Amino-2-methylpropanol neutralisierten Copolymerisates
- 10 bis 76 Gew.-%: Ethanol
- 2 bis 40 Gew.-%: Wasser
- 10 bis 40 Gew.-%: Dimethylether

Die in erfindungsgemäßen Haarfestigungsmitteln enthaltenen Copolymerisate zeichnen sich durch ihre hohe Verträglichkeit mit den unpolaren Treibmitteln in Sprayzubereitungen, insbesondere mit Kohlenwasserstoffen wie Propan oder n-Butan oder ihrem Gemisch aus. Sie weisen eine gute haarfestigende Wirkung auf, ersichtlich an den hohen Werten für die Curl-Retention, die hierbei meist über 80 % liegen. Außerdem zeichnen sich die erfindungsgemäßen Haarfestigungsmittel dadurch aus, daß sie das Haar praktisch nicht verkleben.

Vor allem aber weisen die erfindungsgemäßen Haarfestigungsmittel bei den anwendungstechnischen Eigenschaften, die eingangs bei den Mitteln des Standes der Technik als verbesserungsbedürftig bezeichnet wurden, hervorragende Ergebnisse auf. Sie sind in Alkoholen wie Ethanol oder Isopropanol und in Gemischen dieser Alkohole mit Wasser klar löslich. Die Klarheit der Lösungen bleibt auch erhalten, wenn die Lösungen in Standard-Sprayformulierungen zusammen mit Treibmitteln wie Dimethylether eingesetzt werden. Die erfindungsgemäßen Haarfestigungsmittel sind einwandfrei aus dem Haar auswaschbar. Mit ihnen behandeltes Haar weist eine erhöhte Geschmeidigkeit und einen angenehmen natürlichen Griff auf. Die Festigungswirkung ist gleichzeitig dabei hoch, so daß prinzipiell eine Senkung der benötigten Menge an Filmbildner in der Haarsprayformulierung möglich ist.

Zur gezielten Einstellung der Eigenschaften von Haarfestigungsmitteln kann es von Vorteil sein, die erfindungsgemäßen Copolymerisate als Mischung mit weiteren Haarfestigungspolymeren einzusetzen. Als weitere Haarfestigungspolymere kommen handelsübliche Polymere wie Polyvinylpyrrolidone, Copolymere aus Vinylpyrrolidon und Vinylacetat, Copolymere aus Vinylacetat und Crotonsäure, Copolymere aus Vinylacetat, Vinylpropionat und Crotonsäure oder Copolymere auf Acrylat/Acrylamid-Basis in Betracht. Solche Mischungen können die erfindungsgemäßen Copolymere und die weiteren Haarfestigungspolymere im Gewichtsverhältnis 1 zu 99 bis 99 zu 1 enthalten.

### Beispiele

Herstellungsvorschriften für die Beispiele 1 bis 15 und die Vergleichsbeispiele A bis D

### Beispiel 2 (Emulsionspolymerisation)

Aus 1 g Natriumlaurylsulfat, 6,7 g eines handelsüblichen nichtionischen Emulgators, 100 g Wasser, 1,3 g Ethylhexylthioglycolat, 60 g Methacrylsäure, 210 g tert.-Butylacrylat und 30 g Ethylacrylat stellte man eine Emulsion her, die im Zulaufverfahren in ein Polymerisationsgefäß, das 500 g Wasser enthielt, im Laufe der Polymerisation innerhalb von ca. 2 bis 4 Stunden bei ca. 75 bis 85°C gegeben wurde. Der Initiator, 1 g Natriumpersulfat gelöst in 100 g Wasser, wurde ebenfalls im Laufe der Polymerisation kontinuierlich zugefahren. Zur Senkung des Restmonomerengehalts wurde anschließend mit einem Redoxinitiator (tert.-Butylhydroperoxid/Ascorbinsäure) versetzt und nachpolymersiert.

Die Herstellung der Beispiele 1, 3 bis 11 und A bis D erfolgte analog zu Beispiel 2.

### Beispiel 12 (Lösungspolymerisation)

Eine Mischung aus 105 g tert.-Butylacrylat, 30 g Ethylacrylat, 15 g Acrylsäure, 0,5 g tert.-Butylperpivalat und 25 g Ethanol wurde auf 75°C erwärmt. Nach dem Anspringen der Polymerisation, erkennbar an einer Viskositätserhöhung, wurden gleichzeitig eine Mischung aus 945 g tert.-Butylacrylat, 270 g Ethylacrylat, 135 g Acrylsäure, und 270 g Ethanol und eine Lösung von 6,0 g tert.-Butylperpivalat in 90 g Ethanol im Laufe von 6 Stunden zugegeben, wobei die Temperatur bei schwachem Sieden auf 77 bis 80°C gehalten wurde. Anschließend wurde eine Lösung von 3,0 g 2-5-Dimethyl-2,5-di(tert.-butylperoxy)hexan in 400 g Ethanol zugegeben, wonach der Reaktionskessel druckfest verschlossen, auf 130°C aufgeheizt und 3 Stunden bei dieser Temperatur gehalten wurde. Der Polymerisationsgehalt der erhaltenen Lösung wurde mit ca. 550 g Ethanol auf 50 Gew.-% eingestellt. Das Terpolymerisat hatte einen K-Wert von 32,5 (gemessen in 1,0 %iger ethanolischer Lösung bei 25°C).

Die Herstellung der Beispiele 13 und 14 erfolgte analog zu Beispiel 12.

### Beispiel 15 (Suspensionspolymerisation)

70 g tert.-Butylacrylat, 12 g Ethylacrylat und 18 g Methacrylsäure wurden nacheinander in 250 g Wasser, das 0,9 g Polyacrylsäure vom Molekulargewicht 250.000, 1 g eines handelsüblichen Emulgators auf Basis eines Paraffinsulfonatgemisches mit einer mittleren Kettenlänge von 14 bis 15 C-Atomen und 0,3 g 2-Mercaptoethanol als Regler enthielt, unter Rühren auf 70°C erwärmt. Anschließend wurden 0,02 g tert.-Butyl-per-2-ethylhexanoat in kleinen Mengen portionsweise zugegeben. Nach Zugabe der letzten Portion Initiator wurde noch 3,5 Stunden lang bei 90°C auspolymerisiert. Anschließend wurde bei 100°C Wasserdampf durchgeblasen, bis innerhalb von 4 Stunden ca. 300 g Wasser überdestilliert waren, wobei restliches Monomeres vollständig entfernt wurde. Die erhaltene Polymersuspension wurde abfiltriert und zur Entfernung feinster ungelöster Partikel mit Wasser gewaschen und im Luftstrom bei Temperaturen von ca. 50°C getrocknet, wobei ein rieselfähiges Pulver mit durchschnittlichen Teilchengrößen von 0,3 bis 1,5 mm erhalten wurde. Der K-Wert betrug 40 (gemessen 1 Gew.-% in Ethanol).

### Anwendungstechnische Eigenschaften

Die nachfolgende Tabelle zeigt die Werte für die Zusammensetzung, die Curl-Retention, die Auswaschbarkeit, die Klarlöslichkeit, den Griff und die Festigungswirkung der Beispiele 1 bis 15 und der Vergleichsbeispiele A bis D.

Die Curl-Retention ist ein Maß für die haarfestigende Wirkung unter extremen klimatischen Bedingungen. Sie wird im Modellversuch an Haarlocken gemessen, die durch eine übliche Wasserwelle an ca. 15 cm langen Haaren erzeugt und mit der jeweiligen Sprayzubereitung aus 10 cm Entfernung 4 sec lang besprüht worden sind. Nach einer Verweilzeit von 5 Stunden der aufgehängten Locken in einer Klimakammer bei 25°C und 90 % relativer Luftfeuchtigkeit wird die relative Verformung (Aufweitung) der Locken, bezogen auf ihre ursprüngliche Form, bestimmt. Ein hoher Wert bedeutet eine hohe Festigungswirkung, d.h. bei 100 % bliebe die ursprüngliche Form vollständig erhalten.

Die Auswaschbarkeit des Polymeren aus dem Haar, der Griff (das Anfühlen des behandelten Haares) und die Festigungswirkung (die Stärke der Festigung des Haares am Modellkopf) wurden jeweils mit Noten von 1 bis 5 beurteilt;
dabei bedeutet:

| | |
|---|---|
| 1 = sehr gut | 4 = schlecht |
| 2 = gut | 5 = sehr schlecht |
| 3 = noch gut | |

Die Klarlöslichkeit des Polymers wurde in einer Mischung aus Ethanol, Wasser und Polymer im Gew.-Verhältnis von 55:40:5 wie folgt beurteilt:

| | |
|---|---|
| + = klar löslich | - = trübe |

Alle Messungen und Benotungen wurden jeweils mit einer üblichen Standard-Sprayformulierung bestimmt, in der das Copolymerisat zu 100 % mit 2-Amino-2-methylpropanol neutralisiert war.

### Erklärungen zur Tabelle

- TBA: = tert.-Butylacrylat
- MAS: = Methacrylsäure
- AS: = Acrylsäure
- EA: = Ethylacrylat
- TBAA: = N-tert.-Butylacrylamid

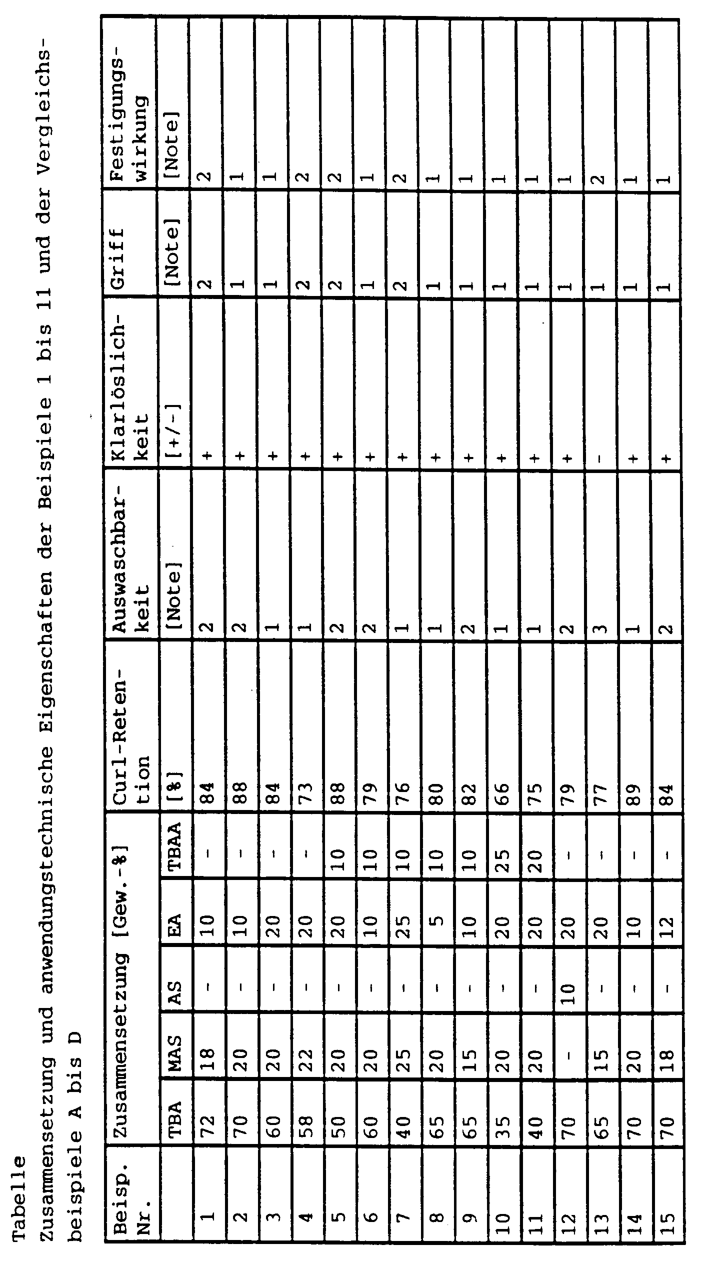

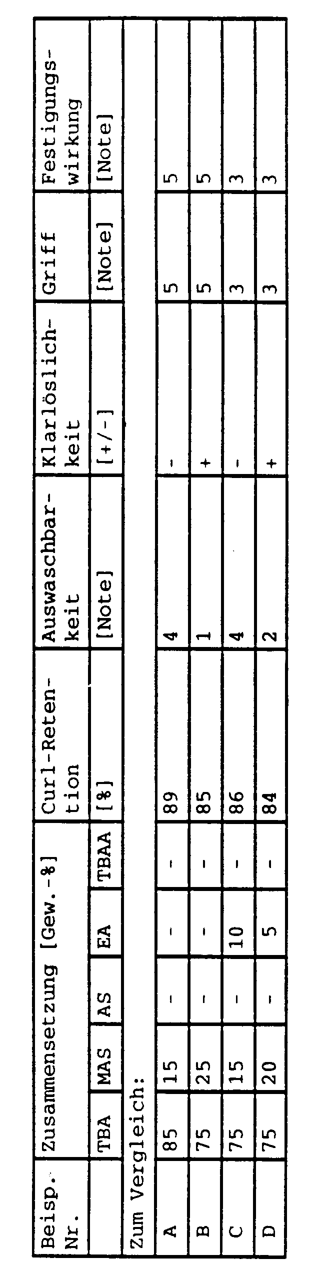

## Patentansprüche

1. Haarfestigungsmittel, enthaltend als Filmbildner Copolymerisate auf der Basis von tert.-Butylacrylat oder tert.-Butylmethacrylat mit einem K-Wert von 10 bis 50, erhältlich durch radikalische Polymerisation von
A) 30 bis 72 Gew.-% tert.-Butylacrylat oder tert.-Butylmethacrylat oder einer Mischung hieraus als Monomerem A,
B) 10 bis 28 Gew.-% Acrylsäure oder Methacrylsäure oder einer Mischung hieraus als Monomerem B und
C) 0 bis 60 Gew.-% eines radikalisch copolymerisierbaren Monomeren oder einer radikalisch copolymerisierbaren Monomerenmischung als Monomerem C, wobei mindestens eines der Monomeren C ein Homopolymerisat mit einer Glastemperatur kleiner als 30°C liefert,
wobei die Carboxylgruppen der Copolymerisate teilweise oder vollständig neutralisiert sind.

2. Haarfestigungsmittel nach Anspruch 1, bei denen das Monomere C ein C₁- bis C₁₈-Alkylacrylat oder -methacrylat oder eine Mischung aus einem C₁- bis C₁₈-Alkylacrylat oder -methacrylat und einem N-C₁- bis -C₁₈-Alkylacrylamid oder -methacrylamid ist.

3. Haarfestigungsmittel nach Anspruch 1 oder 2, in denen der Filmbildner aus
A) 50 bis 72 Gew.-% des Monomeren A,
B) 12 bis 25 Gew.-% des Monomeren B und
C) 3 bis 38 Gew.-% des Monomeren C
aufgebaut ist.

4. Haarfestigungsmittel nach den Ansprüchen 1 bis 3, in denen der Filmbildner aus
A) tert.-Butylacrylat als Monomerem A,
B) Methacrylsäure als Monomerem B und
C) Ethylacrylat oder einer Mischung aus Ethylacrylat und N-tert.-Butylacrylamid als Monomerem C
aufgebaut ist.

5. Haarfestigungsmittel in Form von Sprayzubereitungen, enthaltend neben hierfür üblichen Lösungsmitteln und gegebenenfalls Treibmitteln 0,1 bis 20 Gew.-% eines Copolymerisates gemäß den Ansprüchen 1 bis 4.

6. Haarfestigungsmittel gemäß einem der Ansprüche 1 bis 5, enthaltend zusätzlich weitere übliche für die Haarverfestigung geeignete Polymere.

7. Verwendung der Copolymerisate gemäß den Ansprüchen 1 bis 4 als Filmbildner in Haarfestigungsmitteln.

## Claims

1. A hairsetting composition comprising as film-formers copolymers based on tert-butyl acrylate or tert-butyl methacrylate having a K value of from 10 to 50 which are obtainable by free-radical polymerization of
A) from 30 to 72% by weight of tert-butyl acrylate or tert-butyl methacrylate or a mixture thereof as monomer A,
B) from 10 to 28% by weight of acrylic acid or methacrylic acid or a mixture thereof as monomer B, and
C) from 0 to 60% by weight of a free-radically copolymerizable monomer or of a free-radically copolymerizable monomer mixture as monomer C, where at least one of the monomers C produces a homopolymer having a glass transition temperature of less than 30°C,
the carboxyl groups of the copolymers being partially or completely neutralized.

2. A hairsetting composition as claimed in claim 1, in which the monomer C comprises a C₁-C₁₈-alkyl acrylate or methacrylate or a mixture of a C₁-C₁₈-alkyl acrylate or methacrylate and an N-C₁-C₁₈-alkylacrylamide or -methacrylamide.

3. A hairsetting composition as claimed in claim 1 or 2, in which the film-former is composed of
A) from 50 to 72% by weight of monomer A,
B) from 12 to 25% by weight of monomer B, and
C) from 3 to 38% by weight of monomer C.

4. A hairsetting composition as claimed in any of claims 1 to 3, in which the film-former is composed of
A) tert-butyl acrylate as monomer A,
B) methacrylic acid as monomer B, and
C) ethyl acrylate or a mixture of ethyl acrylate and N-tert-butylacrylamide as monomer C.

5. A hairsetting composition in the form of a spray formulation comprising, in addition to solvents customary for such formulations and, if desired, propellants, from 0.1 to 20% by weight of a copolymer as in any one of claims 1 to 4.

6. A hairsetting composition as claimed in any one of claims 1 to 5, additionally comprising other customary polymers which are suitable for setting hair.

7. The use of a copolymer as in any of claims 1 to 4 as a film-former in a hairsetting composition.

## Revendications

1. Agent fixateur pour cheveux ou fixateur capillaire, contenant, à titre d'agents filmogènes, des copolymères à base d'acrylate de tert-butyle ou de méthacrylate de tert-butyle, d'une valeur K de 10 à 50, que l'on peut obtenir par la polymérisation radicalaire de
A) 30 à 72% en poids d'acrylate de tert-butyle ou de méthacrylate de tert-butyle, ou d'un mélange de ceux-ci, à titre de monomère A,
B) 10 à 28% en poids d'acide acrylique ou d'acide méthacrylique, ou d'un mélange de ceux-ci, à titre de monomère B, et
C) 0 à 60% en poids d'un monomère copolymérisable par voie radicalaire, ou d'un mélange de monomères copolymérisables par voie radicalaire à titre de monomère C, où au moins l'un des monomères C donne un homopolymère avec une température de transition vitreuse inférieure à 30°C,
où les radicaux carboxyle des copolymères sont partiellement ou totalement neutralisés.

2. Fixateur capillaire suivant la revendication 1, dans lequel le monomère C est un acrylate ou un méthacrylate d'alkyle en C₁ à C₁₈, ou un mélange d'un acrylate ou d'un méthacrylayte d'alkyle en C₁ à C₁₈ et d'un N-alkyle(C₁ à C₁₈) acrylamide ou -méthacrylamide.

3. Fixateur capillaire suivant la revendication 1 ou 2, dans lequel l'agent filmogène est constitué de
A) 50 à 72% en poids du monomère A,
B) 12 à 25% en poids du monomère B, et
C) 3 à 38% en poids du monomère C.

4. Fixateur capillaire suivant les revendications 1 à 3, dans lequel l'agent filmogène est constitué
A) d'acrylate de tert-butyle à titre de monomère A,
B) d'acide méthacrylique à titre de monomère B, et
C) d'acrylate d'éthyle ou d'un mélange d'acrylate d'éthyle et de N-tert-butylacrylamide à titre de monomère C.

5. Fixateur capillaire sous la forme de préparations du type spray, contenant, outre les solvants habituels à cette fin et éventuellement des propulseurs, de 0,1 à 20% en poids d'un copolymère suivant l'une quelconque des revendications 1 à 4.

6. Fixateur capillaire suivant l'une quelconque des revendications 1 à 5, contenant complémentairement d'autres polymères usuels convenant à la fixation des cheveux.

7. Utilisation des copolymères suivant l'une quelconque des revendications 1 à 4 à titre d'agents filmogènes dans des fixateurs capillaires.
